Europäisches Patentamt

European Patent Office

Office européen des brevets

(11) Publication number: **0 267 690**
A1

# EUROPEAN PATENT APPLICATION

(21) Application number: 87309074.0

(51) Int. Cl.⁴: **G01N 33/564**

(22) Date of filing: **14.10.87**

(30) Priority: **16.10.86 US 919445**

(43) Date of publication of application:
**18.05.88 Bulletin 88/20**

(84) Designated Contracting States:
**AT BE CH DE ES FR GB GR IT LI LU NL SE**

(71) Applicant: **VASOCOR**
**2438 Wyandotte Street**
**Mountain View California 94013(US)**

(72) Inventor: **Calenoff, Emanuel**
**1824 Oak Creek Drive 319**
**Palo Alto, California 94304(US)**

(74) Representative: **Harrison, David Christopher**
**et al**
**MEWBURN ELLIS & CO 2/3 Cursitor Street**
**London EC4A 1BQ(GB)**

(54) **Immune complex assay.**

(57) The method of this invention for determining the presence of atherosclerotic plaque immune complex in a serum sample comprises separating immune complexes from the serum and treating them with reagents to identify the presence of atherosclerotic plaque antigen and/or anti-(atherosclerotic plaque) antibody in the immune complexes. The immune complexes can be separated by a reagent which selectively isolates only atherosclerotic plaque immune complexes, and then the presence of an immune complex in the selected isolate is confirmed. Alternatively, the immune complexes can be separated by a general non-selective procedure, and the presence of atherosclerotic plaque immune complexes in the isolate can be determined using specific binding reagents. Immune complex isolation can be achieved by precipitation techniques or by insolubilizing the complex on an insoluble support to which a binding agent is adhered.

Kits containing reagents for these procedures are also an aspect of this invention.

EP 0 267 690 A1

## IMMUNE COMPLEX ASSAY

This invention relates to an immunoassay method and reagents for determining the presence of immune complexes in patient serum. One embodiment of this invention relates to a method for diagnosing atherosclerosis by determining the presence of atherosclerotic plaque immune complexes in patient serum.

Immune complexes are formed by the interaction of the complement proteins with antigen-antibody complexes and/or with antigen complexes, and complement fixation exerts it effects on cell membranes, causing lysis of some cells and functional aberrations in others, e.g., degranulation of mast cells with release of histamine, increased permeability of small blood vessels, directed migration of polymorphonuclear leukocytes, increased phagocytic activity by leukocytes and macrophages, and bacteriolysis. Presence of immune complexes is thus associated with immune defensive systems.

The antigen-antibody immune complex thus comprises an antigen conjugated with an antibody selectively binding therewith, products of complement interactions with the antibody-antigen complex, and humoral elements such as antiidiotype antibodies, rheumatoid factors, fibronectin.

The endothelium located between the blood and arterial tissue serves as a barrier against the accumulation of blood components in the vascular wall. Formation of atherosclerotic lesions in the endothelium is associated with major coronary disease and stroke, and the causes and detection of such lesions has been intensely investigated.

Endothelial injury, believed to be an initial step in the formation of the atherosclerotic lesions, can be potentially caused by hemodynamic strain, hypercholesterolemia, hypertension and immune complex disease. Endothelial injury leads to intimal thickening, cell proliferation, cholesterol accumulation and formation of connective tissue fibers. IgG and complement factor C3 accumulation in injured endothelial cells and nonendothehelialized intima has been indicated. Mononuclear phagocytes and other cells of the immune system derived from blood have also been found to constitute a part of the cell population in atherosclerotic lesions. The mechanisms leading to the ultimate plaque composition are as yet unknown.

A wide variety of soluble proteins have been extracted from human atherosclerotic plaque, including IgA, IgG, IgM, BIC(C3), $\alpha_1$-antitrypsin, $\alpha_2$-macroglobulain, fibrinogen, albumin, LDL, HDL, $\alpha_1$-acid glycoprotein, $\beta_2$-glycoprotein, transferrin and ceruloplasmin. The diseased intima was also found to contain a small amount of tissue-bound IgG, IgA and BIC. Hollander, W. et al, *Atherosclerosis.* 34:391-405 (1979). Other scientists have reported IgG in the lesions and adjacent endothelial tissue. Parums, D. et al, *Atherosclerosis.* 38:211-216 (1981), Hansson, G. et al, *Experimental and Molecular Pathology.* 34:264-280 (1981), Hansson, G. et al, *Acta Path. Microbiol. Immunol. Scand. Sect. A.* 92:429-435 (1984). However, the origin, function and binding properties of the immunoglobulins in the atherosclerotic and associated tissue is still a mystery. Anti-low density lipoprotein (LDL) autoantibodies has been reported to be higher in patients of vascular disease, suggesting that they are associated in some way with arteriosclerotic manifestations. However, no causal relationship between these autoantibodies and arteriosclerotic plaque has been established. Szondy, E. et al, *Mechanisms of Aging and Development.* 29:117-123 (1985). Wagner, W., "Proteoglycan structure and function as related to atherosclerosis", ANNALS OF NEW YORK ACADEMY OF SCIENCES. Vol. 454, New York: New York Academy of Sciences pp 52-68 (1985) reported evidence that certain monomers of proteoglycans in atherosclerotic plaque were different from those in adjacent normal aorta.

I have discovered that atherosclerosis is or includes characteristics of an autoimmune disease, atherosclerotic plaque contains plaque specific antigen, and antibodies which bind selectively with this antigen are present in the serum of patients with atherosclerotic disease. In my copending applications Serial No. 846,401 filed March 31, 1986 and Serial No. 871,811 filed June 20, 1986, I have described methods and reagents for determining serum levels of antigens and antibodies which are specific to atherosclerotic plaque. In my copending application Serial No. 919,445 filed October 16, 1986, I reported my finding that serum levels of immune complexes containing atherosclerotic plaque specific antigens and antibodies are often elevated and can be detected.

This application is directed to an improved method for measuring serum levels of immune complexes in serum samples.

A number of immunoassays have been developed for determining the presence and amount of a wide variety of antigenic and non-antigenic materials in diverse body fluids and tissues. Total immunoglobulin and IgE immunoassays are described in U.S. patents 3,720,760 and 4,444,879. IgG allotype immunoassays are described in Russian Patent 649,433. ELISA immunoassays are described by Maggio, et al, ENZYME-IMMUNOASSAY. Boca Raton: CRC Press pp 172-176 (1980).

A method for determining surfactant-antisurfactant immune complexes in serum from infants has been described by Strayer, D. et al, *A.J.P.* 122:353-361 (1986). Antigen determination in immune complexes by a modified staphylococci binding assay and Western blot analysis is described by McDougal, J. et al, *Clinical Immunology and Immunopathology.* 38:184-197 (1986). The solubility of immune complexes are increased by the bonding of the Fc portion of immune complexed antibodies with complement products C4b and C3b, Peterson, I. et al, *Complement.* 2:97-110 (1985).

U.S. Patent 4,343,734 describes the isolation of a protein which appeared to be associated with calcium deposition in the body, for example advanced calcified atherosclerosis, valve calcification, prosthesis calcification. The protein was characterized as containing a γ-carboxyglutamic acid (Gla) group, and this group was monitored in separation procedures of the patent to identify the fractions containing the protein. No protein specific antibody having been successfully prepared, the only marker available to the patentee was the presence of Gla in the fraction. This amino acid is widespread throughout the body in cardiac valves and aortas, and in circulating proteins such as prothrombin, clotting factors VII, IX, and X, Protein C and Protein S, and it does not provide any useful diagnostic assay tool. The paper corresponding to this patent was retracted, and basis for the patent appears to be have been disproven.

EPO application 85402359.5 discloses a monoclonal antibody generated from artificially induced atherosclerotic-like lesions in rabbits which are reported to cross-react with human atherosclerotic plaque lesions. Data indicating the degree of selectivity of the binding was not reported.

The atherosclerotic plaque antigens and atherosclerotic plaque antibodies used in the methods and reagents of this invention are associated with atherosclerotic plaque at all stages, including non-calcified stages, and do not selectively bind with Gla containing proteins.

The improved method of this invention for determining the presence of atherosclerotic plaque immune complex in serum comprises separating atherosclerotic plaque antigen and antibody containing immune complexes from the serum and determining the level of immune complexing elements in the separated material. In one method according to this invention, the serum is contacted with anti-(atherosclerotic plaque antigen) reagent antibody for a time sufficient to permit binding of immune complex containing atherosclerotic plaque antigen in the serum with the anti-(atherosclerotic plaque antigen) reagent antibody. Disease antigen bound thereto which is a component of an immune complex is determined by identifying the presence and/or level of one or more immune complexing elements in the separated material. The immune complexing elements which are detected are selected from the group of complement proteins, fibronectin, antibodies such as plaque antigen binding antibody or antiidiotype antibody, rheumatoid factor, and fibronectin, and is preferably C3b and/or C4b complement protein. The anti-(atherosclerotic plaque antigen) reagent antibody can be bound to an insoluble support, and the insoluble support can be contacted with serum to permit atherosclerotic plaque immune complex antigen conjugation with the anti-(atherosclerotic plaque antigen) reagent antibody. The presence and amount of the immune complex bound to the insoluble support is then determined. In a preferred embodiment, the separated immune complex is contacted with a labeled anti-C4b antibody and/or a labeled anti-C3b antibody for a time sufficient to permit conjugation to occur, and the label conjugated with the insoluble support is determined. Enzyme labels can be developed by reaction with a substrate which yields a physically detectable enzymatic reaction product such as a chromophore or fluorophore.

In another embodiment of the method of this invention, an anti-(human antibody) antibody is bound to an insoluble support, and the insoluble support is contacted with patient serum to bind human antibodies in serum to the insoluble support. The presence of atherosclerotic plaque antigen or anti-(atherosclerotic plaque) antibody in the immune complexes is determined. The anti-(human antibody) antibody can be bound to an insoluble support and contacted with serum to permit the antibody to bind with human antibody in the immune complexes. The presence of atherosclerotic plaque antigen in the insolubilized complex can be determined with labeled anti-(atherosclerotic plaque) antibody. The presence of anti-(atherosclerotic plaque) antibody in the complex can be determined with labeled antiidiotype antibodies which bind preferentially with the binding sites of anti-(atherosclerotic plaque) antibodies.

In another embodiment of the method of this invention, the serum is contacted with anti-(immune complexing element) antibody such as an anti-complement antibody for a time sufficient to permit binding of the immune complexes with the antibody. The presence of atherosclerotic plaque antigen or anti-(atherosclerotic plaque antibody in the immune complexes is then determined. The anti-complement antibody can be bound to an insoluble support and contacted with serum to permit the antibody to bind with complement in the immune complexes. The presence of atherosclerotic plaque antigen in the insolubilized complex can be determined with labeled anti-(atherosclerotic plaque) antibody. The presence of anti-(atherosclerotic plaque) antibody in the complex can be determined with labeled antiidiotype antibodies which bind preferentially with the binding sites of anti-(atherosclerotic plaque) antibodies.

In a still further embodiment of this invention, the serum is treated with polyethylene glycol (PEG) to precipitate immune complexes. The complexes are then solubilized by the addition of a dispersing solution such as a PBS buffer, and the complexes disassociated by electrophoresis or use of chaotropic agents. The resolved proteins are transblotted to a binding support such as nitrocellulose. The presence of atherosclerotic plaque antigen on the support can be determined with labeled anti-(atherosclerotic plaque) antibody. The presence of anti-(atherosclerotic plaque) antibody on the insoluble support can be determined with labeled antiidiotype antibodies which bind preferentially with the binding sites of anti-(atherosclerotic plaque) antibodies and/or labeled atherosclerotic plaque antigen.

A still further aspect of this invention is an immunoassay kit for determining the presence of atherosclerotic plaque immune complex in serum. The kit comprises a labeled anti-(immune complexing element) antibody and preferably C1q$^g$, C4b or C3b antibody or a labeled antiidiotype antibody which binds preferentially with a binding site of an anti-(atherosclerotic plaque) antibody. It also includes a means for insolubilizing immune complex in the serum selected from an immune complex precipitating surfactant, or an insoluble support to which anti-complement antibody, anti-(atherosclerotic plaque) antibody or an antiidiotype antibody which binds preferentially with the binding site of an anti-(atherosclerotic plaque) antibody is bound.

The method of this invention for determining the presence of atherosclerotic plaque plaque immune complex in serum samples comprises separating the immune complexes from the serum either selectively to isolate atherosclerotic plaque immune complexes, or non-selectively and treating them with reagents to identify the presence of atherosclerotic plaque antigen and/or antibody in the immune complexes.

I have discovered that atherosclerotic plaque immune complexes are present in the serum of patients with advanced atherosclerosis. These complexes contain atherosclerotic plaque antigen conjugated with anti-(atherosclerotic plaque) antibody and complement, particularly the solubilizing C3b and C4b complement components. Other complement proteins including C1q$^g$ and complement fragments, antiidiotype antibodies, rheumatoid factor, and fibronectin are found in the immune complex. In these complexes, epitopes of the plaque antigen may be entirely hindered and antibody conjugation therewith entirely blocked by the structure of the immune complex.

The improved method of this invention for determining the presence of atherosclerotic plaque antigen in in serum comprises separating atherosclerotic plaque antigen containing immune complexes from the serum and determining the level of one or more immune complexing elements such as complement proteins C4b, C1g$^g$, and/or C3b in the separated material. In one method according to this invention, the serum is contacted with anti-(athersclerotic plaque antigen) reagent antibody for a time sufficient to permit binding of immune complex containing atherosclerotic plaque antigen in the serum with the anti-(athersclerotic plaque antigen) reagent antibody. This method requires that the atherosclerotic plaque specific epitope be exposed for selective binding by the anti-(athersclerotic plaque antigen) specifically binding antibody. Disease antigen bound thereto which is a component of an immune complex is determined by identifying the presence of an immune complexing element such as C1q$^g$, C4b and/or C3b in the separated material.

An antibody which selectively binds with atherosclerotic plaque antigen is hereinafter referred to as anti-(atherosclerotic plaque) antibody. Also included within the term "antibody" as defined herein antibodies of classes IgG, IgM, IgA, IgD, and IgE, and fragments and hybrid derivatives of antibodies including Fab, and F(ab')$_2$ fragments of antibodies.

The term "plaque antigen" is used hereinafter to denote atherosclerotic plaque antigen, that is, antigen which is uniquely present in substantial concentrations in atherosclerotic plaque and which has one or more epitope which is present either specifically or in increased concentrations in atherosclerotic plaque. Atherosclerotic plaque antigens which are involved in the autoimmune process of the disease are one distinguishing group of antigens within the definition of this term.

The term "atherosclerotic plaque immune complex" and "plaque immune complex", as used herein, is defined to denote complement containing complexes of antigens associated with atherosclerotic plaque and antibodies thereto.

The term "immune complexing elements", as used herein, is defined to mean the antigen-binding antibody, complement compounds and fragments thereof, antiidiotype antibodies, rheumatoid factors, fibronectin and other humoral elements which are found in immune complexes. etc.

In the method of this invention for the diagnosis of atherosclerosis by the measurement of atherosclerotic plaque immune complex in serum, atherosclerotic plaque antigen containing immune complexes are separated from the serum, and the level of immune complexing elements in the separated material is determined. In one method according to this embodiment, the serum is contacted with anti-(atherosclerotic plaque) reagent antibody for a time sufficient to permit binding of atherosclerotic plaque antigen in the serum with the anti-(atherosclerotic plaque) reagent antibody. Plaque antigen bound thereto which is a component of an immune complex is determined by identifying the presence of C4b, C1q⁹, and/or C3b in the separated material. The anti-(atherosclerotic plaque) reagent antibody can be bound to an insoluble support, and the insoluble support can be contacted with serum to permit atherosclerotic plaque immune complex antigen conjugation with the anti-(atherosclerotic plaque) reagent antibody. The presence and amount of the atherosclerotic plaque immune complex bound to the insoluble support is then determined. In a preferred embodiment, the separated immune complex is contacted with a labeled anti-C4b, C1q⁹, antibody and/or a labeled anti-C3b antibody for a time sufficient to permit conjugation to occur, and the label conjugated with the insoluble support is determined. Enzyme labels can be developed by reaction with a substrate which yields a physically detectable enzymatic reaction product such as a chromophore or fluorophore.

In another embodiment of this invention, all of the immune complexes are separate from the serum, such as by binding complement in the complex with anti-complement antibody bound to an insoluble support, by binding antibody with an anti-Ig antibody or protein A bound to an insoluble support, or by precipitation. The presence of atherosclerotic plaque antigen in the insolubilized complex can be determined with labeled anti-(atherosclerotic plaque) antibody. The presence of anti-(atherosclerotic plaque) antibody in the complex can be determined with labeled antiidiotype antibodies which bind preferentially with the binding sites of anti-(atherosclerotic plaque) antibodies.

In still another embodiment of the method of this invention, an anti-(human antibody) antibody is bound to an insoluble support, and the insoluble support is contacted with patient serum to bind human antibodies in serum to the insoluble support. The presence of atherosclerotic plaque antigen or anti-(atherosclerotic plaque) antibody in the immune complexes is then determined. The anti-(human antibody) antibody can be bound to an insoluble support and contacted with serum to permit the antibody to bind with human antibody in the immune complexes. The presence of atherosclerotic plaque antigen in the insolubilized complex can be determined with labeled anti-(atherosclerotic plaque) antibody. The presence of anti-(atherosclerotic plaque) antibody in the complex can be determined with labeled antiidiotype antibodies which bind preferentially with the binding sites of anti-(atherosclerotic plaque) antibodies.

In a still further embodiment of this invention, the serum is treated with polyethylene glycol (PEG) to precipitate immune complexes. Purified complexes are then solubilized using a buffered solution such as PBS, and the complexes disassociated such as by electrophoresis and/or use of chaotropic agents. The resolved components are transblotted to such suitable binding supports such as nitrocellulose. The presence of atherosclerotic plaque antigen on the support can be determined with labeled anti-(atherosclerotic plaque) antibody. The presence of anti-(atherosclerotic plaque) antibody on the insoluble support can be determined with labeled antiidiotype antibodies which bind preferentially with the binding sites of anti-(atherosclerotic plaque) antibodies.

The anti-(atherosclerotic plaque) antibody can be a polyclonal antibody or mixture of polyclonal antibodies obtained by immunizing an animal such as a rabbit, guinea pig, rat or goat with concentrated plaque antigen, removing serum from the immunized animal, and separating the immunoglobulins from the serum, for example by ammonium sulfate precipitation. The principal antibodies useful in the method of this invention are IgG and IgM antibodies, although the IgE and IgA antibodies can also be used if available in sufficient quantity. Alternatively, the anti-(atherosclerotic plaque) antibody can be obtained from a hybridoma derived from a spleen cell of an animal such as a mouse or rat which has been immunized with concentrated plaque antigen. Methods for hybridizing such spleen cells with melanoma cell lines of the respective rodent, selecting clones yielding anti-(atherosclerotic plaque) antibody having the desired selectivity and high binding energy, and producing antibody from the clones are conventional procedures which are well known to a person skilled in the art and are not a part of this invention.

Procedures for obtaining anti-(atherosclerotic plaque) antibody and purified atherosclerotic plaque antigen are described in my copending applications Serial No. 846,401 filed March 31, 1986, Serial No. 871,401 filed March 31, 1986, Serial No. 876,741 filed June 20, 1986 and Serial No. 919,445 filed August , 1986, the entire contents of each of these applications being hereby incorporated by reference in their entireties.

The anti-(atherosclerotic plaque) antibody can be derived directly from atherosclerotic plaque and preferably from purified atherosclerotic plaque antigen by conventional antiserum or monoclonal techniques. Plaque antigen is obtained by processing human atherosclerotic plaque to isolate and purify antigen specific thereto. The plaque can be obtained by processing human aortas or other specific arteries from patients who have died from myocardial infarction, cerebral vascular accident or can be obtained from surgical procedures. The arteries are dissected free from surrounding tissues and fat and rinsed in saline to remove contaminating blood. Segments of plaques are separated and processed.

The plaque areas are removed, cut into small pieces, washed briefly, and homogenized in isotonic solution such as phosphate-buffered saline (PBS). The homogenate repeatedly centrifuged and resuspended in PBS, the elates being collected. The sedimented debris can also be extracted with citrate buffer, acid pH, and the supernatant neutralized and further purified by dialysis. The supernatant can be concentrated by vacuum dialysis and ultrafiltration to yield a concentrate of soluble plaque antigens.

Insoluble plaque antigens can be obtained by digesting portions with different enzymes, extracting the digest with PBS, and concentrating the eluates as described above. The enzyme compositions used can include collagenase, elastase, DNase, hyaluronidase, and neuraminidase. Each digest is centrifuged, concentrated as by ultrafiltration, for example, and dialyzed to yield a concentrated solution of plaque antigens.

Polyclonal anti-(atherosclerotic plaque) antibody can be obtained by immunizing an animal such as a rabbit, guinea pig, rat or goat with concentrated atherosclerotic plaque antigen, removing serum from the immunized animal, and separating the immunoglobulins from the serum, for example by ammonium sulfate precipitation. The principal antibodies useful in the method of this invention are IgG and IgM antibodies, although the IgE and IgA antibodies can also be used if available in sufficient quantity.

Monoclonal anti-(atherosclerotic plaque) antibody can be obtained from anti-plaque antigen by the methods of Glafre and Milstein, *Methods of Enzym.* 73:1 (1981).

In one embodiment of the method of this invention, anti-(atherosclerotic plaque) antibody adhering to an insoluble support is contacted with patient serum for a time sufficient to permit atherosclerotic plaque antigen in the atherosclerotic plaque immune complexes (or immune complexes comprising atherosclerotic plaque antigen) to selectively conjugate with the anti-(atherosclerotic plaque) antibody. The patient serum is removed, and the insoluble support is contacted with an aqueous solution of labeled anti-complement (preferably anti-C4b, anti-C1q$^g$, or anti-C3b) antibody until conjugation with exposed complement epitopes of the atherosclerotic plaque immune complex is effected. The solution of labeled anti-complement antibody is removed from the insoluble support, and the label present on the insoluble support is determined.

Alternatively, assuming the reagent antibody on the insoluble support is a non-human antibody, the insoluble support can be contacted with an aqueous solution of labeled anti-(human antibody) antibody until conjugation of human antibody present in the selectively insolubilized atherosclerotic plaque immune complexes with the labeled anti-(human antibody) antibody is effected. Examples of suitable labeled anti-(human antibody) antibody are labeled anti-(human IgG antibody) antibody, anti-(human IgM antibody) antibody), anti-(human IgA antibody) antibody, or anti-(human IgE antibody) antibody, for example. The label present on the insoluble support is then determined.

In a still further alternative, exposed epitopes of atherosclerotic plaque antigen in the immune complex can be determined by incubating the insoluble support with an aqueous solution of a labeled anti-(atherosclerotic plaque) antibody until conjugation occurs, and determining the label present on the insoluble support.

In another selective separation or insolubilizing method, patient serum is contacted with an insoluble support to which atherosclerotic plaque antigen is adhered for a time sufficient to permit conjugation of the antigen with available binding sites of anti-(atherosclerotic plaque) antibody in the complex. The presence of complement or anti-(atherosclerotic plaque) antibody on the insoluble support is then effected as described above.

Other embodiments of this invention with a non-specific separation step use other proteinaceous binding materials such as anti-complement antibody, anti-Ig (anti-human IgG, IgM, IgA, or IgE) antibody or protein A bound to an insoluble support. These binding materials are well known and commercially available.

As noted above, the presence of the atherosclerotic plaque immune complex on the insoluble support following the initial insolubilization requires a reaction or incubation with a binding agent, preferably a labeled binding agent which will only bind with immune complexes containing a distinctive component of atherosclerotic plaque immune complexes. The specific binding agent can be a labeled atherosclerotic plaque antigen or a labeled antiidiotype antibody which binds specifically with an available binding site of an anti-(atherosclerotic plaque) antibody component of the immune complex. It can also be a labeled anti-(atherosclerotic plaque) antibody for binding with an available plaque epitope of the plaque component of the immune complex.

The term "anti-complement antibody", as used herein, is defined to denote polyclonal and monoclonal antibodies which bind to human complement and complement fragments bound to the antigen or antigen-binding antibody in the immune complex, and preferably to complement epitopes of C4b, C1q$^g$, and/or C3b. Anti-complement antibodies are known for these human complement components and are commercially available. Antibodies binding to human complement components of C4b are preferred because C4b is more stable. Unless C3b binds to a suitable surface, factor H rapidly binds with C3b, leading to rapid cleavage to iC3b (Peterson, I. (supra, p 98)).

The anti-Ig antibody can be an antibody which binds to all classes of human antibody or it can be an antibody which binds to a specific class or subclass (IgG, IgM, IgA, IgE, etc.) which is present in substantial portions in the immune complexes. The preferred anti-IgG antibodies bind with IgG or IgM class of antibodies.

The anti-(atherosclerotic plaque) antibody, anti-complement antibody, anti-human Ig antibody can be bonded to an insoluble support by convention processes. Procedures for binding of antibodies to insoluble supports are described in U.S. patents 3,551,555, 3,553,310, 4,048,298 and RE-29,474, for example. Binding of antibodies to polystyrene by adsorption has been described in U.S. patents 3,646,346 and 4,092,408, for example. Binding of proteinaceous reagents to a variety of insoluble supports has been described in U.S. Patent 3,720,760.

A variety of materials can be used as the insoluble support, the primary consideration being the binding of the antibody or protein A to the surface, the absence of interference with the binding reactions of the reagents or with other reactions which can be employed to determine the presence and extent of the conjugating reaction. Organic and inorganic polymers, both natural and synthetic, can be used as the insoluble support. Examples of suitable polymers include polyethylene, polypropylene, polybutylene, poly(4-methybutylene), butyl rubber, silastic polymers, polyesters, polyamides, cellulose and cellulose derivatives (such as cellulose acetate, nitrocellulose and the like), acrylates, methacrylates, vinyl polymers (such as polyvinyl acetate, polyvinyl chloride, polyvinylidene chloride, polyvinyl fluoride, and the like), polystyrene and styrene graft copolymers, rayon, nylon, polyvinylbutyrate, polyformaldehyde, etc. Other materials which can be used as the insoluble support can the latexes of the above polymers, silica gel, silicon wafers, glass, paper, insoluble protein, metals, metalloids, metal oxides, magnetic materials, semi-conductive materials, cermets and the like. In addition are included substances which form gels, such as proteins such as gelatines, lipopolysaccharides, silicates, agarose, polyacrylamides or polymers which form several aqueous phases such as dextrans, polyalkylene glycols (alkyene with 2 to 3 carbon atoms) or surfactants, e.g. amphophilic compounds such as phospholipids, long chain (12-24 carbon atoms) alkyl ammonium salts and the like.

A preferred diagnostic support of this invention comprises a polystyrene, styrene copolymers such as styrene-acrylonitrile copolymers, or polyolefins such as polyethylene or polypropylene, and acrylate and methacrylate polymers and copolymers. The antibodies and protein A can be bound to the insoluble support by adsorption, ionic bonding, van der Waals adsorption, electrostatic bonding, or other non-covalent bonding, or it can be bound to the insoluble support by covalent bonding. A particularly advantageous support for this procedure comprises a microtiter plate having a plurality of wells. The well surface or plastic cup inserts therein can constitute the antibody or protein A support. If the determination will require the use of fluorometric measurements, the microtiter plate or the well inserts are advantageously opaque to light so that excitation light applied to a well does not reach or influence contents of the surrounding wells.

Procedures for non-covalent bonding are described in U.S. Patent 4,528,267. Procedures for covalently bonding antibodies and protein A to insoluble supports are described by Ichiro Chibata in IMMOBILIZED ENZYMES. Halsted Press: New York (1978) and A. Cuatrecasas, *J. Bio. Chem.* 245:3059 (1970), the entire contents of which are hereby incorporated by reference. The surface can be coated with a protein and coupled with the antibody using procedures described in U.S. Patent 4,210,418 using glutaraldehyde as a coupling agent, for example. In a still further procedure, the well can be coated with a layer having free isocyanate groups such as polyether isocyanate, and application of the antibody in aqueous solution thereto effects the requisite bonding. In a still further procedure, the antibody can be coupled to a hydroxylated material by means of cyanogen bromide as described in U.S. Patent 3,720,760.

Many labeled anti-complement antibodies suitable for use in the method of this invention are also well known, including anti-complement antibodies labeled with enzymes.

Labeled anti-(atherosclerotic plaque) antibodies and methods for their preparation are described in my copending application Serial No. 846,401, filed March 31, 1986. In general, the labels are attached to the antibodies in the same way they are attached to other proteinaceous reagents. Labeled anti-complement antibodies can be prepared from the polyclonal and monoclonal unlabeled antibodies by conventional procedures for attaching labels to proteins.

The labels can be bonded or coupled to the protein reagents by chemical or physical bonding. Ligands and groups which can be conjugated to the antibodies and plaque antigens of this invention include elements, compounds or biological materials which have physical or chemical characteristics which can be used to distinguished the reagents to which they are bonded from compounds and materials in the sample being tested.

Radiolabeled anti-complement antibodies can be used for in vitro diagnostic tests. The specific activity of a tagged antibody depends upon the half-life, isotopic purity of the radioactive label and how the label is incorporated into the antigen or antibody. Table A lists several commonly used isotopes, their specific activities and half-lives. In immunoassay tests, the higher the specific activity, in general, the better the sensitivity.

## TABLE A

| Isotope | Specific Activity of Pure Isotope (Curies/mole) | Half-Life |
| --- | --- | --- |
| $^{14}C$ | $6.25 \times 10^1$ | 5720 years |
| $^3H$ | $2.91 \times 10^4$ | 12.5 years |
| $^{35}S$ | $1.50 \times 10^6$ | 87 days |
| $^{125}I$ | $2.18 \times 10^6$ | 60 days |
| $^{32}P$ | $3.16 \times 10^6$ | 14.3 days |
| $^{131}I$ | $1.62 \times 10^7$ | 8.1 days |

Procedures for labeling proteinaceous antibodies with radioactive isotopes listed in Table A are generally known in the art. Tritium labeling procedures are described in U.S. Patent 4,302,438, for example. Iodinating, tritium labeling and $^{35}S$ labeling procedures especially adapted for antibodies are described by Goding, J.W. MONOCLONAL ANTIBODIES: PRINCIPLES AND PRACTICE. New York: Academic Press pp 124-126 (1983) and the references cited therein. Other procedures for iodinating antibodies are described by Hunter and Greenwood, *Nature* . 144:945 (1962) and David et al, *Biochemistry*. 13:1014-1021 (1974) and in U.S. Patents 3,867,517 and 4,376,110. Examples of other suitable systems, coupling procedures and substrate reactions therewith are disclosed in U.S. Patents Re. 31,006, B1 3,654,090, 4,214,048, 4,289,747, 4,302,438, 4,312,943, 4,376,110 and the references cited therein, for example. Examples of other suitable systems are described by Pesce et al, *Clin.Chem.* 20:353-359 (1974) and Wisdom, G., *Clin.Chem.* 22:1243 (1976).

A list of suitable enzyme classes and specific examples for each class follow:

## TABLE B

| Class | Enzyme Example |
|---|---|
| Hydrolases Carbohydrolases | Amylases |
| Nucleases | Polynucleotidase |
| Amidases | Arginase |
| Purine deaminases | Adenase |
| Peptidases | Aminopolypeptidase |
| Proteinases | Pepsin |
| Esterases | Lipases |
| Iron Enzymes | Catalase |
| Copper Enzymes | Tyrosinases |
| Enzymes containing Coenzymes | Alcohol dehydrogenase |
| Enzymes reducing cytochrome | Succinic dehydrogenase |
| Yellow enzymes | Diaphorase |
| Mutases | Glyoxalase |
| Demolases | Aldolase |
| Oxidases | Glucose oxidase |
| | Horse radish peroxidase |
| Other enzymes | $\beta$-galactosidase |
| | Phosphatases |
| | Phosphorylases |
| | Hexokinases |

A list of suitable enzymes are described in Hawk, et al. PRACTICAL PHYSIOLOGICAL CHEMISTRY, New York: McGraw-Hill pp 306-397 (1954).

Fluorogenic enzymes (enzymes in the presence of which a selected substrate will produce a fluorescent product) are useful labeling moieties. Methods for selectively conjugating enzymes to antibodies without impairing the ability of the anitbody to bind with antigen are well known in the art. Suitable enzymes and procedures for coupling them to antibodies are described by Wilson, M. et al, INTERNATIONAL CONFERENCE IN IMMUNOFLUORESCENCE AND RELATED STAINING TECHNIQUES. W. Knapp et al, editors. Amsterdam: Elsevier pp 215-244 (1978), Sullivan, M. et al, Annals of Clinical Biochemistry. 16:221-240 (1979) and in U.S. Patent 4,190,496, for example. The preferred fluorogenic enzymes and suitable substrates corresponding thereto include horseradish peroxidase for which a suitable substrate is homovanillic acid or 4-hydroxy-3-methoxy-phenylacetic acid, $\beta$-galactosidase for which a suitable substrate is 4-methylumbelliferyl-$\beta$-D-galactoside, alkaline phosphatase for which a suitable substrate is 4-methylumbelliferyl phosphate and other umbelliferyl phosphates such as 4-carboxyumbelliferyl phosphate and umbelliferyl phosphate 4-carboxyalkyl-esters, etc.

Examples of suitable procedures for enzyme labeling the antibody include the use of carbodiimides, dialdehydes, and bifunctional coupling reagents. Linkage of enzymes through amide groups can be achieved by treating the proteins with thionyl chloride, N-hydroxysuccinimide or similar reagents in an anhydrous solvent such as dimethylformamide, dioxane, dimethylsulfoxide, tetrahydrofuran, or the like. Alternative coupling agents include carbodiimides such as 1-ethyl-3-(3-N,N'-dimethylaminopropyl)-

carbodiimide or 1-cyclohexyl-3-(2-morpholinoethyl)carbodiimide methyl-$p$-toluenesulfonate.

The carbohydrate moiety of an enzyme can also be oxidized to an aldehyde and reacted with lysyl amino groups of immunoglobulins to form a Schiffs base. Reduction with sodium borohydride effects a stable linkage of enzyme and antibody. Horseradish peroxidase with antibody can be efficiently linked to immunoglobulins by the method of Wilson, supra.

Fluorescent labeled antibodies can be prepared from standard fluorescent moieties known in the art. Since antibodies and other proteins absorb light having wavelengths up to about 310 nm, the fluorescent moieties should be selected to have substantial absorption at wavelengths above 310 nm and preferably above 400 nm. A variety of suitable fluorescers are described by Stryer, *Science*. 162:526 (1968) and Brand, L. et al, *Annual Review of Biochemistry*. 41:843-868 (1972). The antibodies can be labeled with fluorescent groups by conventional procedures such as those disclosed in U.S. Patents 3,940,475, 4,289,747 and 4,367,110, for example.

One group of fluorescers having a number of the desirable properties described above are the xanthene dyes, which include the fluoresceins derived from 3,6-dihydroxy-9-phenylxanthhydrol and resamines and rhodamines derived from 3,6-diamino-9-phenylxanthydrol and lissanime rhodamine B. The rhodamine and fluorescein derivatives of 9-o-carboxyphenylxanthhydrol have a 9-o-carboxyphenyl group. Fluorescein compounds having reactive coupling groups such as amino and isothiocyanate groups such as fluorescein isothiocyanate and fluorescamine are readily available.

Another group of fluorescent compounds are the naphthylamines, having an amino group in the alpha or beta position. Included among the naphthylamino compounds are 1-dimethylaminonaphthyl-5-sulfonate, 1-anilino-8-naphthalene sulfonate and 2-$p$-toluidinyl-6-naphthalene sulphonate. Other dyes include 3-phenyl-7-isocyanatocoumarin; acridines such as 9-isothiocyanatoacridine and acridine orange; N-[$p$-(2-benzoxazolyl)phenyl]maleimide; benzoxadiozoles such as 4-chloro-7-nitrobenzo-2-oxa-1,3-diazole and 7-($p$-methoxybenzylamino)-4-nitrobenzo-2-oxa-1,3-diazole; stilbenes such as 4-dimethylamino-4'-isothiocyanatostilbene and 4-dimethylanimo-4'-maleimidostilbene; N,N'-dioctadecycloxacarboxyamine-p-toluenesulfonate; pyrenes such as 8-hydroxy-1,3,6-pyrenetrisulfonic acid, 1-pyrenebutyric acid, merocyanine 540, rose bengal, 2,4-diphenyl-3(2H)-furanone, o-phthaldehyde, as well as other readily available fluorescing molecules. These dyes either have active functionalities or such functionalities can be readily introduced.

For example, antibodies can be labeled with fluorochromes by the procedures described by Goding, J. MONOCLONAL ANTIBODIES: PRINCIPLES AND PRACTICE. New York: Academic Press, pp 208-249 (1983). The concentration of fluorochrome is selected according to the table of Goding, p 229. For example, fluorescein isocyanate (1.0 mg/ml) or rhodamine isocyanate (10.0 mg/ml) in DMSO is prepared, and the desired volume (1-10% of total protein solution volume) is added to the protein solution dropwise, with stirring. The reaction proceeds for two hours, shielded from light. The product is purified by gel filtration on SEPHADEX G-25 gel in PBS containing 0.1% NaNO$_3$ to separate the unreacted or hydrolyzed fluorochrome. The absorbence of the conjugate is measured at 280 nm and at its peak in the visible region (495 nm for fluoresceinated antibody and 550 nm for rhodaminated antibody). The fluorochrome to protein ratio is calculated according to the procedure of Goding, supra, p 224-225. Conjugates are stored at 4°C protected from light until use. If the antibody solution concentration is less than 1 mg/ml, BSA is added to the solution to a final concentration of 1 mg/ml.

The anticomplement antibodies used in the method of this invention can be covalently bonded to avidin or biotin in one embodiment of this invention. Suitable binding procedures involve cross-linking through a bifunctional cross-linking agent. Suitable bifunctional compounds are described by Peters, K. et al, *Ann.Rev.Biochim.* 46:523 (1977). Alkyl imidates show a high degree of specificity among the functional groups presented to them by a protein. The reaction is specific for primary amino groups. Examples of suitable coupling reagents include amidoesters such as dimethylmalonimidate, azides such as the acyl azide of tartryl diazide which reacts readily with immuno groups to produce amide linkages. Aryl dihalides (e.g., 1,5-difluoro-2,4-dinitrobenzene, or 4,4'-difluoro-3,3'-dinitrophenyl sulfone, glutaraldehyde, 1-ethyl-3-(3-dimethylaminopropyl)carbodiimide hydrochloride, dimaleinide, mixed anhydride, m-maleamidobenzoyl N-hydroxysucciinimide ester, and other known cross-linking agents.

The foregoing reagents provide essentially irreversible bonds. Bifunctional agents with functional groups such as disulfide or glycol may be used. These provide bonds which can be broken after the cross-linking reaction, if desired. Such reagents include dimethyl 3,3'-dithiobispropionimidate, succinimidyl-propionimidate, N-(3-fluoro-4,6-dinitrophenyl)-cystamine, tartryl diazide, tartryl di(glycylazide) and tartryl di-(epsilon-amino caproylazide).

In other instances, the bonds can be formed directly between the reagents themselves. For example, antibody can be bound to biotin through functional groups on the respective materials. As a specific example, biotin can be treated with periodate and reacted with antibody to give a Schiff base formation without inhibiting the biotin to avidin binding or blocking immunological activity of the antibody.

Known techniques using bifunctional cross-linking agents include the following: (a) a one-step glutaraldehyde linkage, Avrameas, S., *Immunochemistry.* 6:43 (1969); (b) two-step glutaraldehyde linkage, Avrameas, S., *Immunochemistry.* 8:1175 (1971); and (c) dimaleimide linkage, Kato, K. et al, *Euro.J.Biochem.* 62:285 (1966).

Antibodies can be labeled with metallic radionuclides according the procedure of Hnatowich, D. et al. *Journal of Applied Radiation.* 35:554-557 (1984) and Buckley, R et al. *Federation of European Biochemical Societies.* 166:202-204 (1984). In this procedure the antibodies are conjugated with a chelating agent such as diethylenetriamine pentaacetic acid which is capable of forming a chelate with the metallic radionuclide. A suspension of 0.1 mg/ml of the bicyclic anhydride of DTPA (diethylenetriamine pentaacetic acid) is prepared in a dry solvent such as chloroform, ether or dry DMSO. An aliquot is removed to a clean, dry tube sufficient to provide a DTPA to immunoglobulin molar ratio of 1:1 and evaporated under nitrogen. A 10-20 microliter portion of the antibody solution used (10-20 mg/ml) in 0.05M bicarbonate buffer in saline, pH 7.0-7.5 is added to the dry DTPA, and the contents are agitated for 0.5-1.0 min. The coupled protein preparation is diluted to 0.2 ml with the same buffer solution and purified on a 5 cm gel filtration column with SEPHADEX G-50 gel, using a saline eluant. The coupling efficiency is determined before purification by the addition of "chelation-grade": $^{111}$In in 0.5M acetate buffer solution, pH 6.0. Thin layer chromatography is used to separate the DTPA coupled antibody for calculation of the coupling efficiency. The DTPA-coupled antibodies can be stored at 4°C until needed for binding with metallic radionuclides such as $^{111}$In + 3, $^{212}$Bi + 3 and $^{68}$Ga + 3, for example.

In one embodiment of the immunoassay method of this invention, an insoluble support to which anti-plaque antibody is adhered is contacted with patient serum for a sufficient time to permit conjugation of antigen containing immunocomplex in the serum with the anti-plaque antibody on the insoluble support, and then removing the patient serum from the support. The incubation time should be sufficient to permit substantial conjugation to occur, the time being temperature dependent. Suitable incubation times are from 30 to 240 minutes at temperatures within the range of from 16 to 40°C, the preferred contact time being at least 60 minutes at temperatures within the range of from 20 to 26°C.

The residual serum is then removed form the support by use of a rinse solution. Any conventional rinse solution can be used. A preferred rinse solution is described in U.S. Patent 4,528,267. It is an aqueous phosphate buffer solution having a phosphate molarity of from 0.0001 to 0.05, a pH of from 6 to 8 and containing from 0.001 to 0.1 weight percent of non-ionic surfactant. Suitable non-ionic surfactants include polyoxyethylene ethers (BRIJ such as lauryl, cetyl, oleyl, stearyl, and tridecyl polyoxyethylene ethers; polyoxyethylene sorbitans (TWEEN such as polyoxyethylene sorbital monolaurate, monopalmitate, monostearate, monoleate, and trioleates); and other polyoxyethylene ethers (TRITON, for example). A preferred non-ionic surfactant is octylphenoxypolyethoxy ethanol having 40 ethylene oxide units (TRITON X-405, Rohm and Hass Company).

The insoluble support is then contacted with labeled anti-(C4b, C1q$^g$, and/or C3b) complement antibody which will bind with complement on the insoluble support.

A variety of labels have been described above. For purposes of clarity and not by way of limitation, the subsequent steps of the process will be described for anti-complement antibodies which have been labeled with an enzyme, preferably a fluorogenic enzyme. The term "fluorogenic enzyme" is defined herein to refer to an enzyme which will produce a fluorophore product with a suitable substrate.

The enzyme labeled anti-complement antibody is applied to the insoluble support in an aqueous solution. The solution preferably contains suitable salts and buffers to preserve the reactants and facilitate the conjugation reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit labeled anti-plaque antibody to conjugate with exposed plaque antigen epitopes, if any, adhering to the insoluble support. The preferred incubation times and temperatures are as set forth for the conjugation of insolubilized reagent anti-(atherosclerotic plaque) antibody with the atherosclerotic plaque immune complex.

The labeled anti-complement antibody solution is then removed form the insoluble support, and the support is rinsed with a rinse solution such as is describe above, to remove any residual, unconjugated labeled material.

In a third step of this embodiment, the insoluble support is contacted with an aqueous solution of a substrate which undergoes a reaction in the presence of the enzyme to release fluorescent compound into the solution. Suitable substrates and the enzymes which which they can be converted are described in U.S. Patents 4,190,496 and 4,528,267, for example. The support is contacted with an aqueous solution of the substrate containing from $10^{-2}$ to $10^{-10}$ molar concentrates of the substrate. Substrate molar concentrations

of from $10^{-4}$ to $10^{-5}$ are preferred. Preferred additional reagents and buffers in the substrate solution include 2-amino-2-methyl-1-propanol buffer and magnesium chloride, for example.

The substrate solution is incubated with the insoluble support for sufficient time for the reaction yielding the fluorophore to occur. At temperatures of from 18 to 40°C, incubation times of from 5 to 240 minutes can be used. Preferably, the temperature is within the range of from 20 to 26°C, and the incubation time is from 30 to 90 minutes.

The fluorescent level in the solution is then measured. The equipment and procedures for determining the level of fluorescence in the substrate solutions are those conventionally used in the art. The level of fluorescence is a function of the enzyme concentration on the insoluble support which is, in turn, a function of the amount of plaque antigen or plaque antigen immunocomplex in the serum sample. The presence and concentration of the atherosclerotic plaque immune complex can be determined by comparing the fluorescence level of the solution with fluorescence levels obtained with control solutions containing known concentrations of immune complex.

As an alternative, the insoluble support with specific atherosclerotic plaque immune complex conjugated thereto can be incubated with an enzyme labeled anti-(human Ig) antibody or enzyme labeled atherosclerotic plaque antigen for a sufficient time to permit conjugation to occur. The preferred incubation times and temperatures are as set forth above for the incubation of insolubilized reagent anti-(atherosclerotic plaque) antibody with the atherosclerotic plaque immune complex. The conjugation product is then separated, washed and reacted with a substrate solution as described above.

In another embodiment of the method of this invention, the serum is contacted with a reagent which will bind all immune complexes containing complement such as an anti-complement antibody (preferably an anti-C4b, anti-C1q$^g$, or anti-C3b antibody), or with a reagent which will bind all immune complexes not containing complement such as an anti-(human Ig) antibody for a time sufficient to permit binding of the immune complexes with the antibody. The anti-complement or anti-Ig antibody is preferably bound to an insoluble support. The patient serum is then removed from the support. The incubation time should be sufficient to permit substantial conjugation to occur, the time being temperature dependent. Suitable incubation times are from 30 to 240 minutes at temperatures within the range of from 16 to 40°C, the preferred contact time being at least 60 minutes at temperatures within the range of from 20 to 26°C. The residual serum is then removed from the support by use of a rinse solution such as the phosphate buffer solution described above.

The presence of atherosclerotic plaque antigen or anti-(atherosclerotic plaque antibody in the immune complexes is then determined. The presence of atherosclerotic plaque antigen in the insolubilized complex can be determined with labeled anti-(atherosclerotic plaque) antibody. The presence of anti-(atherosclerotic plaque) antibody in the complex can be determined with labeled antiidiotype antibodies which bind preferentially with the binding sites of anti-(atherosclerotic plaque) antibodies.

The labeled reagent is applied to the insoluble support in an aqueous solution. A variety of labels have been described above. For purposes of clarity and not by way of limitation, the subsequent steps of the process will be described for anti-plaque and/or antiidiotype antibodies which have been labeled with an enzyme, preferably a fluorogenic enzyme.

The enzyme labeled antibody is applied to the insoluble support in an aqueous solution. The solution preferably contains suitable salts and buffers to preserve the reactants and facilitate the conjugation reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit labeled anti-plaque antibody to conjugate with exposed plaque antigen epitopes, if any, adhering to the insoluble support, to permit labeled antiidiotype antibodies to conjugate with exposed binding sites of anti-(atherosclerotic plaque) antibody in the immune complex or both. The preferred incubation times and temperatures are as set forth for previously described antibody-antigen reactions.

The labeled reagent solution is then removed form the insoluble support, and the support is rinsed with a rinse solution such as is described above, to remove any residual, unconjugated labeled material. For purposes of clarity and not by way of limitation, the subsequent steps are described for anti-plaque and/or antiidiotype antibodies which have been labeled with an enzyme, preferably a fluorogenic enzyme.

The enzyme labeled antibody is applied to the insoluble support in aqueous solution. The solution preferably contains suitable salts and buffers to preserve the reactants and facilitate the conjugation reaction. For example, the solution can contain bovine serum albumin (BSA), phosphate buffer solution (PBS), and a mild surfactant such as polyoxyethylene sorbitan ester employed in the above-described rinse solution. The incubation is continued for sufficient time to permit labeled anti-plaque antibody to conjugate with exposed plaque antigen epitopes, is any, adhering to the insoluble support, to permit labeled antiidiotype antibodies to conjugate with exposed binding sites of anti-(atherosclerotic plaque) antibody in the immune complex or both. The preferred incubation times and temperatures are as set forth for previously described antibody-antigen reactions.

The labeled reagent solution is then removed from the insoluble support, and the support is rinsed with a rinse solution such as is described above, to remove any residual, unconjugated labeled material.

In a third step of this embodiment, the insoluble support is contacted with an aqueous solution of a substrate which undergoes a reaction in the presence of the enzyme to release fluorescent compound into the solution such as described above with respect to the first described embodiment of this invention. The support is contacted with an aqueous solution of the substrate containing from $10^{-2}$ to $10^{-10}$ molar concentrations of the substrate. Substrate molar concentrations of from $10^{-4}$ to $10^{-5}$ are preferred. Preferred additional reagents and buffers in the substrate solution include 2-amino-2-methyl-1-propanol buffer and magnesium chloride, for example.

The substrate solution is incubated with the insoluble support for sufficient time for the reaction yielding the fluorophore to occur. At temperatures of from 18 to 40°C, incubation times of from 5 to 240 minutes can be used. Preferably, the temperature is within the range of from 20 to 26°C, and the incubation time is from 30 to 90 minutes.

The fluorescent level in the solution is then measured. The level of fluorescence is a function of the enzyme concentration on the insoluble support which is, in turn, a function of the amount of plaque antigen or plaque antigen immunocomplex in the serum sample. The presence and concentration of the atherosclerotic plaque immune complex can be determined by comparing the fluorescence level of the solution with fluorescence levels obtained with control solutions containing known concentrations of immune complex.

In the third embodiment of this invention, the serum is treated with a water-soluble or water-dispersible polyethylene glycol (PEG) having a molecular weight of from 500 to 10,000 and preferably from 1000 to 5000, or an equivalent protein precipitating surfactant to precipitate immune complexes. Purified complexes are then solubilized with a suitable aqueous buffer solution, and the immune complex disassociated with electrophoresis or chaotropic agent. The resolved proteins are transblotted to a protein or other binding support such as nitrocellulose. The presence of atherosclerotic plaque antigen on the support can be determined with labeled anti-(atherosclerotic plaque) antibody. The presence of anti-(atherosclerotic plaque) antibody on the insoluble support can be determined with labeled antiidiotype antibodies which bind preferentially with the binding sites of anti-(atherosclerotic plaque) antibodies. If the labels used with the reagents are chromogenic or fluorogenic enzymes, the above described procedures can be used to obtain a physically detectable stain on the Western blot, selecting a substrate which yields the desired detectable product. Alternatively, the label can be a radiolabel, in which case, the Western blot can be developed by conventional radiographic methods customarily used with Western blot methods.

This invention is further illustrated by the following specific, but non-limiting examples. Temperatures are given in degrees Centigrade and percents as weight percents unless otherwise specified. Examples which are constructively reduced to practice herein are presented in the present tense, and examples representing laboratory experiments previously reduced to practice are presented in the past tense.

## EXAMPLE 1

*Isolating Atherosclerotic Plaque Antigen*

The intimal layer (plaque) is stripped from the medial layer, and washed briefly in 0.9 N Saline. The plaque is cut into small pieces (2 x 2 mm), and the tissue is homogenized in 0.15 M PBS at pH 7.2 at high speed for 30-60 sec at 4°C. The homogenate is centrifuged (2000 x g) at 4°C for 30 min. The supernatant is saved. The homogenizing and centrifuging steps are repeated, pooling the supernatants. Sodium azide in bacteriostatic quantities is added to the pooled supernatant, and the supernatant is stored at 4°C.

## EXAMPLE 2

*Antibody conjugation to Sepharose*

Freeze-dried CNBr-Sepharose 4B powder (Pharmacia) is swelled for 15 minutes in 1 mM HCl. The gel is washed on a sintered glass filter (porosity G-3) with a total of 200 ml of 1 mM HCl per gram of gel (dry wt.) This is done in several aliquots, the supernatant being suctioned off between successive additions.

5 Mg of protein to be coupled per 1 ml of gel is dissolved in Coupling Buffer (0.1 M NaHCO₃, pH 8.3, containing 0.5 M NaCl). The gel is washed with Coupling Buffer, the excess is removed by suction, and the protein solution is mixed with the gel. The mixture is allowed to stand overnight at 4°C without stirring. The gel is then placed in a Blocking Buffer containing 1 M ethanolamine, pH 8.0, for 2 hr at rm temp. The gel is then washed with the Coupling buffer, 0.1 M Acetate Buffer, pH 4.0, containing 0.5 M NaCl, and washed twice with Coupling Buffer. The protein-Sepharose conjugate is now ready for use and can be stored at 4 to 8°C. Cyanogen bromide can be added to the buffer solution as a bacteriostat.

## EXAMPLE 3

*IgG Antibody Adsorption from Plaque Supernatant*

A column is packed with 25 ml of Sepharose gel conjugated to anti-IgG antibody prepared in accordance with the procedure of Example 2, containing a total of about 129 mg of anti-IgG antibody. The column is equilibrated with from 8 to 10 volumes of buffer (0.15 M PBS, pH 7.2), and the plaque homogenate supernatant is then applied to the column.

The flow rate of eluting buffer (0.1 M acetic acid, 0.15 M NaCl, pH 2.3) is preferably 30-60 ml/hr. The eluted fractions containing antibody are collected until peak activity disappears.

## EXAMPLE 4

*IgE Antibody Adsorption from Plaque Supernatant*

The procedure of Example 3 is repeated with a column packed with 7.5 ml of Sepharose gel conjugated to anti-IgE antibody prepared in accordance with the procedure of Example 2. The flow rate of eluting PBS buffer is 30-60, and preferably 37.5 ml/hr.

## EXAMPLE 5

*IgA Antibody Adsorption from Plaque Supernatant*

The procedure of Example 3 is repeated with a column packed with 7.5 ml of Sepharose gel conjugated to anti-IgA antibody prepared in accordance with the procedure of Example 2. The flow rate of eluting PBS buffer is 30-60, and preferably 37.5 ml/hr.

## EXAMPLE 6

*IgM Antibody Adsorption from Plaque Supernatant*

The procedure of Example 3 is repeated with a column packed with 10.0 ml of Sepharose gel conjugated to anti-IgM antibody prepared in accordance with the procedure of Example 2. The flow rate of eluting PBS buffer is 30-60, and preferably 50 ml/hr.

## EXAMPLE 7

*Plaque Antigen Purification*

IgG antibody binding specifically with atherosclerotic plaque obtained in accordance with the procedure of Example 3 is bound to Sepharose in accordance with the procedure of Example 2 to form an affinity column gel binding specifically to plaque antigen.

A column is packed with 25 ml of Sepharose gel conjugated to anti-plaque IgG antibody. The column is equilibrated with 8-10 volumes of buffer (0.15 M PBS, pH 7.2), and plaque solution obtained according to the procedure of Example 1 is applied to the column.

The flow rate of eluting buffer (0.1 M acetic acid, 0.15 M NaCl, pH 2.3) is preferably 30-60 ml/hr. The eluted fractions containing plaque antigen are collected until peak activity disappears, yielding plaque antigen to which the anti-plaque IgG antibody specifically binds.

The column is then washed with 8 to 10 x volumes of buffer (0.15 PBS, pH 7.2), and the antigen eluate is dialyzed against PBS at 40°C, 24-36 hr, with multiple buffer changes.

Repeating this procedure but replacing the gel conjugated with anti-plaque IgG antibodies with gel conjugated with anti-plaque IgA, IgE and IgM antibodies obtained in accordance with the procedures of Examples 4-6, respectively, yields the corresponding plaque fractions to which the antibodies specifically bind.

## EXAMPLE 8

*Polyclonal Anti-Plaque Antibodies*

Polyclonal antiserum against atherosclerotic plaque antigen prepared in accordance with the procedure of Example 7 is elicited in rabbits using the immunization techniques and schedules described in the literature, e.g., Stollar, *Methods of Enzymology.* 70:70 (1980). The antiserum is screened in a solid phase assay similar to that used for monoclonal antibodies, e.g., as described by Lange et al, *Clin.Exp.Immunol.* 25:191 (1976) and Pisetsky et al, *J.Immun.Methods.* 41:187 (1981). The initial screening criterion would be binding to atherosclerotic plaque antigen.

The IgG fraction of the antisera is purified further by affinity chromatography on a column containing a resin on which the anti-plaque antigen is immobilized, following the procedures of Example 3.

## EXAMPLE 9

*Extraction of Highly Specific Plaque Antigens*

Human arterial sections containing significant fibro-fatty atherosclerotic plaque were harvested at autopsy within 6 hr of death and quickly frozen to -80°C. At the time of processing, the arterial samples were thrawed at room temperature and washed three times with 0.01M PBS, pH 7.3, with 0.02% NaN$_3$ - (PBS) to remove blood and other particulates. The atherosclerotic plaque was carefully dissected from surrounding normal appearing artery, and the artery discarded. Significant calcification was dissected away. The remaining fibro-fatty plaque was cut into 2 mm pieces and and added to a two fold volume of cold PBS with 5 uM of the protease inhibitor phenylmethylsulfonyl fluoride (PMSF) added to the PBS. This suspension was homogenized on ice in a VIRTIS homogenizer for 2 min. The homogenized suspension was passed through two layers of loose mesh gauze to remove large particulates. It was then centrifuged at 20,000 rpm for 30 min at 6°C. The plaque supernatant was then carefully removed, and the precipitate was discarded. The protein content of the plaque supernatant was determined by spectrophotometric analysis.

In order to separate and identify the molecular fractions possessing antigens highly specific for the atherosclerotic plaque, the plaque supernatant was fractionated by passing it through a 55 x 200 mm BIO-GEL TSK DEAE-5-PW anion exchange column (Bio-Rad, Richmond, CA) by way of high pressure liquid chromotrography equilibrated with a 20 mM NaH$_2$PO$_4$/Na$_2$HPO$_4$ phosphate buffer (PB). The amount of plaque supernatant loaded on the anion exchange column was sufficient to load 500 mg of total protein. The molecular constituents of the plaque affixed to the anion exchange column were then fractionated by passing through the PB solution with an increasing gradient of PB + 0.5 M NaCl (0 to 100% PB + 0.5 M NaCl running 4 hr) at a 6 ml/min flow rate. Six cc collections were made in each of approximately 240 test tubes.

To determine which of the tubes contained molecules with highly specific epitopes, 100μl from each sample tube was applied to two wells in black IMMULON II microtiter plates (Dynatech, Chantilly, VA). The plates were covered and incubated overnight at 4°C. The following morning, the plates were blocked for 1 hr with 1% BSA in PBS solution at room temperature. The plates were then washed (200 μl/well x 4) with a solution of PBS plus 0.1% TRITON-X-100 (octylphenoxy polyethoxyethanol, Sigma, St. Louis) and 0.05% TWEEN 20 (polyoxylene sorbitan mono-oleate, Sigma). To each of the wells corresponding to each test tube fraction was added 100 μl of the pooled serum from 80-100 patients with severe atherosclerotic diseases diluted 1:100 in 5% BSA in PBS. To the other well was added a 1:100 dilution of serum pooled from 80-100 young males and females under age 20. These diluted sera were incubated for 2 hr, and the plates were washed four times with wash buffer. To each well was then added goat anti-human IgG-alkaline phosphatase conjugate (Tago, Burlingame, CA) diluted 1:2000 in 5% BSA in 0.02 M Tris/NaCl buffer with 0.02% $NaN_3$, 100 $\mu$l/well for 2 hr.

The wells were then washed four times with wash buffer and 100 $\mu$l of 4-methylumbelliferyl phosphate substrate solution (3M Diagnostics, Santa Clara, CA) applied to each well. The plates were read at 5 min intervals with a FLUOROFAST 96 well fluorometer (3M Diagnostics). Each pair of wells corresponding to each test tube fraction were evaluated for the ratio of fluorescent signal between the well having been incubated with pooled atherosclerotic patient serum and the well incubated with pooled sera from young, asymptomatic patients.

Only one cluster of tubes was positive for a signal ratio greater than 3:1. The contents of these tubes were pooled, dialyzed against the PB solution using 3500 dalton SPECTRAPHOR dialysis tubing (Spectrum Medical Industries, Los Angeles, CA) and run through the above anion exchange separation and antigen fraction identification procedure to attain a more pure antigen fraction. Those tubes whose contents possessed antigen activity giving a signal ratio of 4:1 or greater were retained and their contents pooled. The pooled solution was dialyzed against PBS with PMSF and then concentrated in a DIAFLO concentrating system with a 1000 dalton filter (Amicon Div., W.R.Grace, Danvers, Mass.) to attain a protein content of 1 mg/ml, and sufficient sodium azide is added to yield a concentration of 0.03%. The solution was stored at 4°C.

## EXAMPLE 10

*Anti-Plaque Monoclonal Antibody*

Balb/C mice, 7 wks old, were immunized with the atherosclerotic plaque antigen. On day 1, they were injected subcutaneously with plaque antigen in Complete Freunds Adjuvant. On day 16, they were again injected subcutaneously with plaque antigen in Complete Freunds Adjuvant. On Day 23, the mice were test bled, the mouse with the highest antibody titer against the immmizing antigen being chosen for fusion. On Day 40, the mouse was boosted with an intravenous injection of antigen. On Day 44, the mouse was sacrificed.

The spleen from the immunized mouse was made into a cell suspension using the frosted ends of two glass slides. The total number of cells was about $1.97 \times 10^8$. The cells were pelleted and resuspended in 60 ml Dulbecco's Modified Eagles medium (high glucose) (DMEM) with 20 ug/ml (microgram/ml) lipopolysaccharide (LPS), 5% fetal calf serum (FCS). $2 \times 10^6$/ml of spleen cells were then plated out in 6 well culture plates. After 24 hr of LPS stimulation, the cells were fused. A SP2 cell line was grown in culture before fusion, and the cells were used in the growth phase. The LPS stimulated lymphocytes were transfered to 50 ml conical centrifuge tubes, the cells were pelleted by centrifuge at 1000 rmp for 5 min, and combined in one tube with $50 \times 10^6$ SP2 cells.

Cells were washed twice to remove protein with DMEM. After final centrifugation, 2 ml of PEG (polyethylene glycol, Baker 1450, Baker Chemical), 40% was added, and the cells were resuspended and centrifuged for 2 min at 300 rpm, 2 min at 600 rpm and 3 min at 1000 rpm. The supernatant was removed, and the cells were resuspended in 5 ml of DMEM, with no protein. The suspension was centrifuged for 7 min at 1000 rpm.

Cells were then resuspended in 240 ml DMEM, High Glucose with 15 mM Hepes $10^{-4}$ hypoxanthine and 2 ug/ml Azozerine and 20% FCS. Flat bottom tissue culture 96 well plates were previously filled with 150 $\mu$l/well same medium.

100 $\lambda$ of fusion suspension was added to each well. Plates were incubated in 7% $CO_2$ at 37°C in a humidity incubator. The total number of plates were 24. Hybrids were detected on Day 5 and Day 13. 150 $\lambda$ of spent culture supernatant was collected from wells having a growing hybrid. This fusion was plated out to give no more than 20% of the wells with growing hybrids, allowing for easier detection of specific hybrids, initially. The hybrids continued to grow in complete medium, removing the Azozerine after two weeks. As the hybrids were selected, they were expanded into flasks then frozen in liquid hydrogen.

The supernatants collected from wells with growing hybridomas were screened by the ELISA method of Example 3 and the immuno-fluorescence histology method of Example 4, yielding hybridomas yielding antibodies with the binding specificities sought.

## EXAMPLE 11

*ELISA Hybridoma Selection Monoclonal Antibody Screening*

Black IMMULON II microtitre plates were coated with 0.1 ug of plaque antigen fraction derived from HPLC runs carried out in accordance with the procedure of Example 9, diluted in 100 µl PBS, pH 8.5. The plates were incubated at 4°C for 12-18 hr and then blocked with 1% BSA in PBS for 1 hr. The plates were washed four times with PBS wash buffer, and then 100 µl of supernatant from each hybridoma containing well was added to individual wells on the IMMULON II plates. These were incubated for 2 hr and washed four times with the standard Wash Buffer. The Wash Buffer can be prepared by diluting the following concentrate with 50 parts by volume of distilled or deionized water to one part by volume of concentrate. The concentrate is 0.5 wt.% BSA, 0.1 wt.% non-ionic surfactant (TRITON X-405), 17 wt.% sodium chloride, 2 wt.% sodium azide, 0.05 M sodium phosphate, the pH adjusted to 7.4. Then 100 µl of goat anti-mouse IgG/alkaline phosphatase conjugate (Tago, Burlingame, CA) diluted in 0.02 M Tris/NaCl with 5% BSA was added and incubated for 2 hr. The plates were washed four times, and 100 µl of 4 MUP substrate solution (methylumbelliferyl phosphate, 3M Diagnostics, Santa Clara, CA) was added. The plates were read at intervals in a FLUOROFAST 96 well fluorometer (3M Diagnostics). Hybridomas showing positive in this assay were expanded and retested.

Those monoclonal antibodies that remained positive were then screened for their ability to capture antigens in atherosclerotic plaque to which the antibodies in human sera reacted. This was done by purifying each monoclonal antibody reacting positive in the ELISA screen using an HPLC sizing column, BIO-SIL TSK-400 (Bio-Rad, Richmond, CA) and eluting the various size fractions with 0.1 M $KH_2PO_4/K_2HPO_4$, pH 7.0, at 2 ml min flow rate. The pooled fractions containing each monoclonal antibody were dialyzed against PBS. To determine which antibodies capture the specific antigens, 1 ug/well of each antibody diluted in PBS, pH 8.5, was applied to the black IMMULON II plates and incubated overnight. The plates were then blocked with 1% BSA in PBS for 1 hr and then washed four times with PBS wash buffer. To each plate was added 100µl/well of the crude plaque supernatant used in the HPLC runs. This was incubated for 2 hr. The plates were washed four times. Then 100 µl of 1:2, 1:4, 1:8, 1:16, 1:32, 1:64, 1:128, 1:256, 1:512, and 1:1024 dilutions in 5% BSA/PBS of the atherosclerotic patient pooled serum were applied to duplicate wells, and similar dilutions of pooled negative control serum from asymptomatic, young patients were added to the duplicate set of wells. Those were incubated for 2 hr, washed four times, and then 100 µl of goat anti-human IgG alkaline phosphatase diluted in 5% BSA/Tris were added to each well. After a 2 hr incubation, the plates were washed four times, and 100 µl of 4-MUP was added to each well. The plates were read in the microtiter plate fluorometer. One antibody appeared to capture antigen as its fluorescence signal sequence displayed a classical sigmoid curve indicative of antigen-antibody interaction when the scores of the various dilutions of the atherosclerosis positive sera were plotted out (human IgG concentration on a vertical axis, serum dilution on a horizontal axis. This was corroborated by differences in signal (high) registered by the atherosclerosis positive patient sera versus the normal patient's sera (low).

## EXAMPLE 12

*Antibody Coated Micotiter Plate*

100 µl of prepared dilutions of anti-(atherosclerotic plaque) antibody prepared in accordance with the procedures of Example 8 (or Example 10) are applied to the surface of IMMULON II microtiter plates (Dynatech). The coating solution dilutions are selected to be from 1-5 micrograms/well but can be varied up or down depending upon the selection of other reagents and immunoassay procedures to be followed. The plates are tapped gently and to insure the coating solution covers the bottom of each well completely. The wells are incubated at 4°C overnight in a covered, humidified box.

The coating solution is discarded, and 200 µl of 1% BSA in PBS is added per well. The wells are then incubated at rm temp for 1 hr in a humidity box, and the BSA solution is removed. The wells are then washed with 200 µl of Wash Buffer (PBS, 0.5% TWEEN, and 0.2% sodium azide), and stored in a humidity box at 4°C until use.

## EXAMPLE 13

*Enzyme Labeled Complement Antibody*

Anti-complement antibody binding to human complement C4b (Sigma) is conjugated with alkaline phosphatase following the modified procedure of O'Sullivan, M. et al. *Analytical Biochem.* 100:100 (1979), the entire contents of which are hereby incorporated by reference.

In like manner, alkaline phosphatase labeled anti-(human complement) antibody which binds specifically with C3b, C3bi, C3d, C3g and c1q complement is prepared.

## EXAMPLE 14

*Fluorescence Immunoassay Procedure for Atherosclerotic Plaque Immune Complex*

To each microtiter plate coated with a different concentration of anti -(atherosclerotic plaque) antibodies prepared in accordance with the procedure of Example 13 is applied 100 μl/well of the serum sample of the patient being tested, diluted 1:100. The plates are covered to prevent drying and incubated for 2 hr. The sera is removed, and the plate is washed 3 times with Wash Buffer.

100μl/well of a mixture of anti-(human complement C4b, C3b, C3d, C3g, C1q, and/or 3Cbi) antibodies, conjugated with alkaline phosphatase in accordance with the procedure of Example 13 is applied to each well, and the plates are covered to prevent drying and incubated 2 hr. The enzyme labeled antibody solution is removed, and the plates are washed 3 times with Wash Buffer.

100 μl/well of 4-methylumbelliferyl phosphate solution (3M Diagnostics Systems) is then applied to the well. The microtiter plates are then read in a fluorometer (3M Diagnostics) every 10 minutes until the first 4096 reading or 1 hr is reached. The readings are continued every 30 minutes for another 2 hr.

## EXAMPLE 15

*Enzyme Labeled Anti-Plaque Antibody*

Anti-plaque antibody prepared in accordance with the procedures of Example 8 or 10 is conjugated with alkaline phosphatase following the modified procedure of O'Sullivan, M. et al, *Analytical Biochem.* 100:100 (1979), the entire contents of which are hereby incorporated by reference.

Horseradish peroxidase is conjugated to anti-plaque antibody in accordance with the procedure of Nygren, H. et al, *Medical Biology.* 57:187-191 (1979) as follows. Horseradish peroxidase (HRP, Type II or Type VI, Sigma) is dissolved in 0.05 M carbonate: bicarbonate buffer, pH 9.5, containing 0.25% glutaraldehyde (GA, Polaron). After 2 hr at room temperature, the excess GA is separated from the GA-HRP on a Sephadex G-25 column (0.7 12 cm, Pharmacia) equilibrated with 0.15 M NaCl. The GA-HRP complex, is, in a second step, mixed with the antibody in 0.05 M carbonate: bicarbonate buffer, pH 9.5, containing 0.15 M NaCl, at different IgG:HRP ratios for 16-64 hr at 4°C. The reaction is stopped by the addition of lysine to a final concentration of 0.02 M.

## EXAMPLE 16

*Enzyme Labeled Anti-idiotype Antibody*

Anti-idiotype antibody is conjugated with alkaline phosphtase following the modified procedure of O'Sullivan, M. et al, supra. 100:100 (1979), the entire contents of which are hereby incorporated by reference.

Horseradish peroxidase is conjugated to anti-idiotype antibody in accordance with the procedure of Nygren, H. et al, supra. Horseradish peroxidase (HRP, Type II or Type VI, Sigma) is dissolved in 0.05 M carbonate: bicarbonate buffer, pH 9.5, containing 0.25% glutaraldehyde (GA, Polaron). After 2 hr at room temperature, the excess GA is separated from the GA-HRP on a Sephadex G-25 column (0.7 12 cm, Pharmacia) equilibrated with 0.15 M NaCl. The GA-HRP complex is, in a second step, mixed with the antibody in 0.05 M carbonate: bicarbonate buffer, pH 9.5, containing 0.15 M NaCl, at different IgG: HRP ratios for 16-64 hr at 4°C. The reaction is stopped by the addition of lysine to a final concentration of 0.02 M.

## EXAMPLE 17

*Anti-complement Antibody Coated Microtiter Plate*

The procedure of Example 12 is repeated replacing the anti-(atherosclerotic plaque) antibody with a mixture of equal parts of anti-complement antibodies binding specifically with human complement C4b and C3b residues in immunocomplexes.

## EXAMPLE 18

*Fluorescence Immunassay Procedure for Atherosclerotic Immunocomplex*

To each microtiter plate coated with anti-complement antibody prepared in accordance with the procedures of Example 17 is applied 100 μl/well of the serum sample of the patient being tested, diluted from 1:1 to 1:1000. The plates are covered to prevent drying and incubating for 2 hr. The sera is removed, and the plate is washed 3 times with Wash Buffer.

100 μl/well of alkaline phosphatase conjugated anti-plaque antibody prepared in accordance with the procedure of Example 16 or alkaline phosphatase conjugated antiidiotype antibody prepared in accordance with the procedure of Example 17 is applied to each well, and the plates are covered to prevent drying and incubated 2 hr. The enzyme labeled antibody solution is removed, and the plates are washed 3 times with Wash Buffer.

100 μl/well of 4-methylumbelliferyl phosphate solution (3M Diagnostics Systems) is then applied to the well. The microtiter plates are then read in a fluorometer (3M Diagnostics) every 10 minutes until the first 4096 reading or 1 hr is reached. The readings are continued every 30 min for another 2 hr.

As an alternate procedure, a mixture of 100 μl/well of a 1:1 mixture of alkaline phosphatase conjugated anti-plaque antibody prepared in accordance with the procedure of Example 16 and alkaline phosphatase conjugated antiidiotype antibody prepared in accordance with the procedure of Example 16 is applied to each well, and the above procedure is repeated.

## EXAMPLE 19

*Western Blot Method*

The procedure followed is an adaptation of the isolation procedure described by McDougal, J. et al. *Clin.Immuno.Immunopath.* 38:184-197 (1986). 50 μl of test serum, 150 μl of EDTA buffer, and 1.0 ml of 6.0% PEG (5% final PEG concentration) are added to a 10 x 75 mm glass tube. After overnight incubation at 4°C, the tubes are centrifuged at 1000g for 20 min. The supernate is aspirated, and the pellet is suspended in 5% PEG and centrifuged again. The supernate is completely aspirated, and the pellet is suspended in 200 μl of PBS and allowed to stand at rm temp for 15-20 min with agitation or until the precipitate is dissolved. The supernatant is prepared for Western blot analysis by adding 10% (v/v) glycerol and bromophenol blue (5% v/v of a 0.5 mg/ml solution), and the sample is heated at 65°C for 30 min. A chaotropic agent can be used to protect labile antigen.

SDS-dissociated proteins are resolved by the discontinuous, gradient SDS-PAGE system of Neville and Grossman, METHODS OF ENZYMOLOGY. (S. Fleischer and L. Packer, eds.) Vol. 32, New York: Academic Press, p 92; electrophoretically transferred to nitrocellulose by the method of Towbin et al, *Proc.Natl.Acad.Sci.USA.* 76:4350 (1979); and detected with enzyme-linked antibodies following the procedure of Tsang et al, METHODS IN ENZYMOLOGY. (J. Langone and H. van Vunakis, eds) Vol. 92, New York: Academic Press, p377 (1983). Electrophoresis was done on 5-μ sample volumes on 3.3-20% gradient acrylamide gels. PAGE-resolved proteins are electrophoretically transferred to nitrocellulose sheets (60 V for 3 hr, 4°C). Sheets are washed at 45°C with three changes (15 min each) of prewarmed PBS containing 0.3% (v/v) polyoxyethylene sorbitan monolaurate (TWEEN-20, Sigma). Sheets are then incubated overnight at 4°C on a horizontal rotary platform with horseradish peroxidase-conjugated anti-(atherosclerotic plaque) antibody prepared in accordance with the procedure of Example 16, and other sheets are similarly incubated overnight with horseradish peroxidase-conjugated antiidiotype antibody prepared in accordance with the procedure of Example 18. In each incubation, the labeled antibodies are diluted in PBS-0.3% (v/v) TWEEN-20. Sheets are washed 3 times at rm temp with PBS-TWEEN-20. Color reactions are developed

with 3,3′-diaminobenzidine and $H_2O_2$. If indirect staining is desired, the sheets are incubated for 2 hr at rm temp with a HPO-anti-Ig and washed again before developing the color reaction.

Dye-conjugated proteins (Bethesda Research Laboratories) can be used as molecular markers. The leading edges of the dye conjugated markers are calibrated against unconjugated markers, and the molecular weights indicated beside the blots are derived from the calibration results. The bands are compared with the bands of similar sheets prepared with reagent anti-(atherosclerotic plaque) antibody and with reagent purified atherosclerotic plaque antigen.

## Claims

1. A method for determining the presence of atherosclerotic plaque immune complex in a serum sample comprising

a) contacting the sample with an atherosclerotic plaque immune complex specific binding reagents for a time sufficient to permit conjugation of the atherosclerotic plaque immune complex with the atherosclerotic plaque immune complex specific binding agent, and

b) determining atherosclerotic plaque immune complex conjugated with the atherosclerotic immune complex specific binding agent.

2. The method of Claim 1 comprising

a) contacting the sample with an insoluble support to which anti-(atherosclerotic plaque) antibody is adhered for a time sufficient to permit atherosclerotic plaque immune complex conjugation with the anti-(atherosclerotic plaque) antibody to occur to bind atherosclerotic plaque antigen having exposed plaque specific epitopes, and removing residual sample from the support;

b) contacting the insoluble support from step (a) with labeled immune complex binding reagent selected from the group consisting of labeled anti-complement antibody, a labeled anti-(human Ig) antibody selected from the group consisting of anti-(human IgG) antibody, anti-(human IgM) antibody, anti-(human IgA) antibody, and anti-(human IgE) antibody, and anti-fibronectin antibody for a time sufficient to permit conjugation of the labeled immune complex binding reagent with the immune complex present on the insoluble support, and removing unconjugated labeled immune complex binding reagent from the support; and

c) determining the label present on the insoluble support.

3. The method of Claim 2 wherein the label is an enzyme and the label present on the insoluble support is determined by contacting the insoluble support with a substrate which in the presence of the enzyme, produces a physically detectable product.

4. The method of Claim 1 wherein the anti-complement antibody is an anti-(human complement) antibody.

5. The method of Claim 4 wherein the anti-complement antibody is binds preferentially with complement C4b, C3b, C3d or C3g.

6. The method of Claim 5 wherein the anti-complement antibody binds preferentially with complement C4b.

7. The method of Claim 2 wherein the labeled immune complex binding reagent is a labeled anti-(human Ig) antibody selected from the group consisting of anti-(human IgG) antibody and anti-(human IgM) antibody.

8. A method for determining the presence of atherosclerotic plaque immune complex in a serum sample comprising

a) separating immune complex from the sample, and

b) determining the presence of atherosclerotic plaque antigen or anti-(atherosclerotic plaque) antibody in the immune complex.

9. The method of Claim 8 wherein the sample is contacted with a non-specific immune complex binding agent selected from the group consisting of anti-complement antibody or anti-(human Ig) antibody for a time sufficient to permit binding of the immune complex binding agent with the immune complex.

10. The method of Claim 9 comprising

a) contacting the sample with an insoluble support to which the non-specific immune complex binding agent is bound, and removing residual sample from the insoluble support;

b) contacting the insoluble support with a labeled atherosclerotic plaque immune complex binding agent selected from the group consisting of anti-(atherosclerotic plaque) antibody, atherosclerotic plaque antigen and a labeled antiidiotype antibody which binds preferentially with a binding site of anti-(atherosclerotic plaque) antibody, for a time sufficient to permit binding of the labeled atherosclerotic plaque immune complex binding agent with the immune complex to occur, and removing residual unconjugated, labeled atherosclerotic plaque immune complex binding agent from the insoluble support; and

c) determining the label present on the insoluble support.

11. The method of Claim 10 wherein the label is an enzyme and the label present on the insoluble support is determined by contacting the insoluble support with a substrate which in the presence of the enzyme, produces a physically detectable product.

12. The method of Claim 9 wherein the non-specific immune complex binding agent is anti-complement antibody.

13. The method of Claim 12 wherein the anti-complement antibody binds preferentially with complement C4b, C3b, C3d or C3g.

14. The method of Claim 8 comprising

a) separating the immune complex from serum components,

b) dissociating the immune complex to release any anti-(atherosclerotic plaque) antibody or atherosclerotic plaque antigen from other immune complex components,

c) separating the dissociated immune complex components by gel electrophoresis and binding a portion thereof to a binding support.

d) contacting the binding support with a labeled specific atherosclerotic plaque immune complex binding agent selected from the group consisting of anti-(atherosclerotic plaque) antibody, atherosclerotic plaque antigen and antiidiotype antibody which binds preferentially with a binding site of anti-(atherosclerotic plaque) antibody, for a time sufficient to permit antigen-antibody binding to occur, and removing residual unconjugated, labeled antibody from the protein binding support; and

c) determining the label present on the protein binding support.

15. The method of Claim 14 wherein the label is an enzyme and the label present on the protein binding support is determined by contacting the insoluble support with a substrate which in the presence of the enzyme, produces a physically detectable product.

16. The method of Claim 15 wherein the label is a radiolabel.

17. An immunoassay kit for determining the presence of atherosclerotic plaque immune complex in serum comprising

a) a labeled specific immune complex binding agent selected from the group consisting of anti-(atherosclerotic plaque) antibody, atherosclerotic plaque antigen, and antiidiotype antibody which binds preferentially with a binding site of an anti-(atherosclerotic plaque) antibody, and

b) a means for insolubilizing immune complex in the serum selected from an immune complex precipitating surfactant, or an insoluble support to which is bound, an immune complex binding agent is adhered, the immune complex binding agent consisting of anti-complement antibody, protein A, anti-(human Ig) antibody, anti-(atherosclerotic plaque) antibody, atherosclerotic plaque antigen, and antiidiotype antibody which binds preferentially with the binding site of an anti-(atherosclerotic plaque) antibody.

18. The immunoassay kit of Claim 17 wherein the immune complex precipitating surfactant is water-dispersible polyethylene glycol having a molecular weight of from 500 to 10,000.

19. The immunoassay kit of Claim 17 wherein the means for insolubilizing immune complex in the serum is an insoluble support to which an immune complex binding agent is adhered.

20. The immunoassay kit of Claim 19 wherein the immune complex binding agent is an anti-(human complement) antibody bound thereto.

21. The immunoassay kit of Claim 20 wherein the anti-(human complement) antibody binds preferentially with complement C4b, C3b, C3d or C3g.

22. The immunoassay kit of Claim 21 wherein the anti-(human complement) antibody binds preferentially with complement C4b.

23. The immunoassay kit of Claim 19 wherein the immune complex binding agent is an anti-(human Ig) antibody.

23. The immunoassay kit of Claim 17 wherein the labeled anti-(atherosclerotic plaque) antibody or a labeled antiidiotype antibody has an enzyme label, and the kit further includes a substrate which in the presence of the label products a physically detectable product.

24. An immunoassay kit for determining the presence of atherosclerotic plaque immune complex in serum comprising

a) a primary binding reagent which specifically binds with atherosclerotic immune complex selected from the group consisting of anti-(atherosclerotic plaque) antibody, atherosclerotic plaque antigen, and antiidiotype antibody which specifically binds with a binding site of anti-(atherosclerotic plaque) antibody; and

b) a labeled immune complex binding agent selected from the group consisting of anti-(human) complement antibody, anti-(human Ig) antibody, and a secondary binding agent which specifically binds with atherosclerotic plaque immune complex selected from the group consisting of anti-(atherosclerotic plaque) antibody, atherosclerotic plaque antigen, and the antiidiotype antibody which binds preferentially with a binding site of an anti-(atherosclerotic plaque) antibody, wherein the secondary binding agent is not the same as the binding agent.

25. The immunoassay kit of Claim 24 wherein the secondary binding agent is an anti-(human) complement antibody.

26. The immunoassay kit of Claim 25 wherein the anti-(human) complement antibody binds preferentially with complement C4b, C3b, C3d, or C3g.

27. The immunoassay kit of Claim 26 wherein the anti-(human) complement antibody binds preferentially with complement C4b.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 008 432 (BEHRINGWERKE AKTIENGESELLSHAFT) * Page 2, line 18 - page 3, line 3; page 4, lines 27-37; page 5, lines 30-39, page 16, line 1 - page 17, line 29 * | 1-3,10, 11,15, 16,19, 23 | G 01 N 33/564 |
| X | WO-A-8 201 593 (D. PARRAT) * Page 5, lines 6-21; page 8, lines 17-24; page 13, line 3 - page 15, line 3 * | 1-5,9-13,15, 16,19-21,23, 25,26 | |
| X | WO-A-8 102 469 (SCRIPPS CLINIC & RESEARCH FOUNDATION) * Page 3, table I; page 5, line 24 - page 6, line 17; page 7, lines 3-15; page 9, table II; page 32, lines 9-29; page 40, lines 2-36 * | 1-5,8-17,19-21,23-26 | |
| X | EP-A-0 155 141 (TEIJIN LTD) * Page 2, lines 9-27; page 3, lines 14-26; page 4, lines 24-34; page 19, line 3 - page 20, line 14 * | 1,2,4-6,12,13, 20-22, 25-27 | TECHNICAL FIELDS SEARCHED (Int. Cl.4) G 01 N |
| Y | EP-A-0 034 301 (BEHRINGWERKE AKTIENGESELLSCHAFT) * Page 1, lines 4-17; page 2, line 14 - page 3, line 12; page 4, lines 22-31; page 8, claims * | 1-27 | |
| Y | GB-A-2 043 888 (THE WELLCOME FOUNDATION) * Page 1, line 32-39; page 2, lines 10-36; page 3, lines 6-8 * | 1-27 | |

-/-

The present search report has been drawn up for all claims

| Place of search | Date of completion of the search | Examiner |
|---|---|---|
| THE HAGUE | 04-02-1988 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................
& : member of the same patent family, corresponding document

EPO FORM 1503 03.82 (P0401)

European Patent
Office

**EUROPEAN SEARCH REPORT**

Application Number

EP  87 30 9074

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 90, 1979, page 56, abstract no. 97699d, Columbus, Ohio, US; S. GERO et al.: "Circulating immune complexes in human and experimental vascular diseases", & INT. CONF. ATHEROSCLER., [PROC.] 1977 (Pub. 1978), 575-81 | 1-27 | |
| Y | CHEMICAL ABSTRACTS, vol. 104, 1986, page 443, abstract no. 32840f, Columbus, Ohio, US; R. VLAIEU et al.: "Immunohistochemical localization of the terminal C5b-9 complement complex in human aortic fibrous plaque", & ATHEROSCLEROSIS (Shannon, Irel.) 1985, 57(2-3), 163-77 | 1-27 | |

TECHNICAL FIELDS SEARCHED (Int. Cl.4)

The present search report has been drawn up for all claims

| Place of search | Date of completion of the search | Examiner |
|---|---|---|
| THE HAGUE | 04-02-1988 | VAN BOHEMEN C.G. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)